# EUROPEAN PATENT APPLICATION

(11) **EP 3 616 607 A1**
(43) Date of publication of application: **04.03.2020**
(21) Application number: 18792289.3
(22) Date of filing: 15.03.2018
(51) Int. Cl.: A61B 5/0408, A61B 5/0404, A61B 5/0478

(54) **BIOSENSOR**

(30) Priority: 28.04.2017 JP 2017090539; 07.03.2018 JP 2018040596
(71) Applicant: Nitto Denko Corporation, Ibaraki-shi Osaka 567-8680 (JP)
(72) Inventor: YOSHIOKA, Ryoma, Ibaraki-shi Osaka 567-8680 (JP); TOYODA, Eiji, Ibaraki-shi Osaka 567-8680 (JP); TAKEMURA, Keiji, Ibaraki-shi Osaka 567-8680 (JP); MORI, Shigeyasu, Ibaraki-shi Osaka 567-8680 (JP)
(74) Representative: Hoffmann Eitle
(86) International application number: PCT/JP2018/010199
(87) International publication number: WO 2018/198569

(57) **Abstract**

A biosensor includes a pressure-sensitive adhesive layer for attaching to a surface of a living body, a substrate layer disposed on an upper face of the pressure-sensitive adhesive layer and having a stretching property, a probe disposed on the lower face of the pressure-sensitive adhesive layer, and an electronic component mounted on the substrate layer so as to be connected to the probe, wherein a total thickness of the pressure-sensitive adhesive layer and the substrate layer is 1 µm or more and less than 100 µm.

## Description

### TECHNICAL FIELD

The present invention relates to a biosensor.

### BACKGROUND ART

Conventionally, a biosensor that is attached to a surface of the living body, senses the living body, and is wearable has been known.

For such a biosensor, for example, Patent Document 1 has proposed a physiological monitoring device including a housing having rigidity and accommodating a print circuit substrate assembly, and a flexible wing that extends from the housing (for example, see Patent Document 1).

In the physiological monitoring device described in Patent Document 1, the wing is a flexible body including an electrode, an upper substrate layer and bottom substrate layer sandwiching the electrode, and a pressure-sensitive adhesive layer positioned below the bottom substrate layer. The thickness of the upper substrate layer and bottom substrate layer is adjusted so that the entire thickness of the flexible body is between 0.1 mm and 1.0 mm.

In Patent Document 1, the physiological monitoring device including the flexible body is attached (pressure-sensitive adhesion) to the skin, which allows for precise measurement of biosignals for a long period of time continuously.

### Citation List

### Patent Document

Patent Document 1: Japanese Translation of PCT International Application Publication No. 2016-504159

### SUMMARY OF THE INVENTION

### PROBLEM TO BE SOLVED BY THE INVENTION

However, recently, there is a demand for a monitoring device that is not only capable of continuous measurement of biosignals for a long period of time, but also capable of achieving comfortable wearability.

However, the following may be of some concern. With the configuration described in Patent Document 1, in which many semiconductor chips and electronic components are mounted on a rigid wire substrate (PCBA), the thickness of the device housing is large, and not in close contact with the skin surface, and therefore movement of the body at the time of continuous wearing catches the wing to be easily removed, and as a result, the motion artifact may increase.

The present invention provides a biosensor that can decrease discomfort in wearing for a living body.

### MEANS FOR SOLVING THE PROBLEM

The present invention (1) includes a biosensor including a pressure-sensitive adhesive layer for attaching to a surface of a living body, a substrate layer disposed on an upper face of the pressure-sensitive adhesive layer and having a stretching property, a probe disposed on the lower face of the pressure-sensitive adhesive layer, and an electronic component mounted on the substrate layer so as to be connected to the probe, wherein a total thickness of the pressure-sensitive adhesive layer and the substrate layer is 1 µm or more and less than 100 µm.

The present invention (2) includes the biosensor described in (1), wherein the pressure-sensitive adhesive layer has a thickness of 10 µm or more and 95 µm or less.

The present invention (3) includes the biosensor described in (1) or (2), wherein the electronic component has a thickness of 1 µm or more and 1000 µm or less.

The present invention (4) includes the biosensor described in any one of (1) to (3), wherein the electronic component has a flat area of 0.001 mm² or more and 10 mm² or less.

### Effects of the Invention

In the biosensor, a total thickness of the pressure-sensitive adhesive layer and the substrate layer is thin, i.e., 1 µm or more and less than 100 µm, and therefore even when the biosensor is attached to the surface of the living body, discomfort in wearing of a living body can be sufficiently decreased.

With the biosensor, the total thickness of the pressure-sensitive adhesive layer and the substrate layer is small, and therefore production costs per one biosensor can be decreased, and therefore, biosensor can be disposable.

### BRIEF DESCRIPTION OF THE DRAWINGS

[FIG. 1] FIG. 1 shows a plan view of a wearable biosensor in one embodiment of the biosensor of the present invention.
[FIG. 2] FIG. 2A and FIG. 2B show cross sectional views of the wearable biosensor shown in FIG. 1: FIG. 2A is a cross sectional view along line A-A, and FIG. 2B is a cross sectional view along line B-B.
[FIG. 3] FIG. 3A to FIG. 3D are process diagram of production of the wearable biosensor shown in FIG. 2A, FIG. 3A illustrating a step of preparing a substrate layer and a wire layer, FIG. 3B illustrating a step of bonding the pressure-sensitive adhesive layer and the substrate layer, FIG. 3C illustrating a step of forming an opening and preparing a probe member, FIG. 3D illustrating a step of inserting the probe member to the opening, and forming a connector.
[FIG. 4] FIG. 4 is a perspective view seeing the probe-containing sheet from the bottom, cutting out the second release sheet partially.
[FIG. 5] FIG. 5 is a perspective view illustrating a production process of the probe member, the upper side figure showing a perspective view seeing from the lower side, and the lower side figure showing a perspective view seeing from the upper side.
[FIG. 6] FIG. 6A to FIG. 6C are exploded perspective views of the probe member, FIG. 6A showing the probe member, FIG. 6B showing the connecter, and FIG. 6C showing the opening of the wearable biosensor at a longitudinal one end portion.
[FIG. 7] FIG. 7A and FIG. 7B are cross sectional views of the wearable biosensor in a modified example of the one embodiment, FIG. 7A illustrating an embodiment in which the upper portion of the probe is embedded in the pressure-sensitive adhesive layer, and FIG. 7B illustrating an embodiment in which the probe is not embedded in the pressure-sensitive adhesive layer and is projected from the pressure-sensitive adhesive layer.

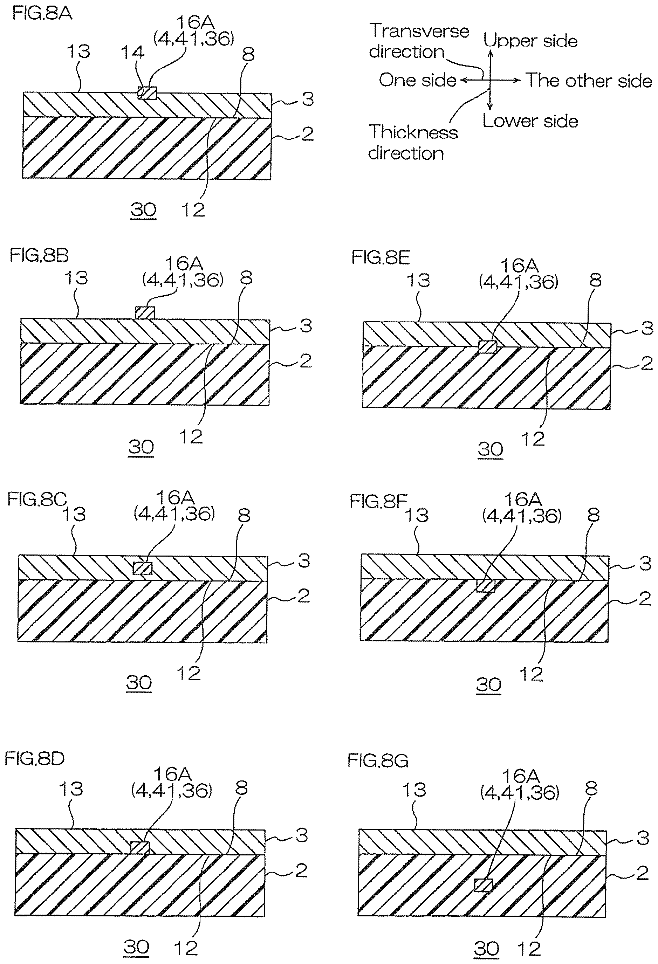

[FIG. 8] FIG. 8A to FIG. 8G are cross sectional views of the wearable biosensor in a modified example of the one embodiment, FIG. 8A illustrating an embodiment in which the lower portion of the wire layer is embedded in the substrate layer, FIG. 8B illustrating an embodiment in which the wire layer is not embedded in the substrate layer and is projected from the substrate layer to the upper side, FIG. 8C illustrating an embodiment in which the wire layer is not exposed from the substrate layer and the wire layer is embedded in the substrate layer, FIG. 8D illustrating an embodiment in which the wire layer is embedded in the substrate layer so as to be exposed from the substrate lower face, FIG. 8E illustrating an embodiment in which the wire layer is embedded in both of the pressure-sensitive adhesive layer and the substrate layer, FIG. 8F illustrating an embodiment in which the wire layer is embedded in the pressure-sensitive adhesive layer so as to be exposed from the adhesive upper face, and FIG. 8G illustrating an embodiment in which the wire layer is embedded in the pressure-sensitive adhesive layer without being exposed from the pressure-sensitive adhesive layer.
[FIG. 9] FIG. 9 shows exploded perspective views of the wearable biosensor in a modified example of the one embodiment (connecter is a substantially half ring shape in plan view).
[FIG. 10] FIG. 10 shows exploded perspective views of the wearable biosensor in a modified example of the one embodiment (embodiment in which the connecter has an upper end portion substantially half ring shape in plan view and a plurality of first strip extending toward lower side).
[FIG. 11] FIG. 11A to FIG. 11C show perspective views of the probe member in the wearable biosensor in a modified example of the one embodiment, FIG. 11A illustrating an embodiment in which the connecter has slits, FIG. 11B illustrating an embodiment in which the connecter has second strips, and FIG. 11C illustrating an embodiment in which the connecter has a mesh portion.
[FIG. 12] FIG. 12 shows a cross-sectional view of the wearable biosensor in a modified example of the one embodiment (probe has a substantially flat plate shape).
[FIG. 13] FIG. 13 shows a cross-sectional view of the wearable biosensor in a modified example of the one embodiment (probe has a substantially solid pillar shape).
[FIG. 14] FIG. 14 shows a plan view of the wearable biosensor in a modified example of the one embodiment (connecter has a substantially rod (needle) pillar shape).
[FIG. 15] FIG. 15 shows a cross sectional view along line A-A of the wearable biosensor shown in FIG. 14.
[FIG. 16] FIG. 16 shows a cross-sectional view of the wearable biosensor in a modified example of the one embodiment (connecter has a substantially rod (needle) shape, and the probe has a substantially plate shape).
[FIG. 17] FIG. 17A and FIG. 17B show another modified example of the wearable biosensor shown in FIG. 15, FIG. 17A illustrating an embodiment in which the electrical conductive pressure-sensitive adhesive layer is provided on the probe lower face, and FIG. 17B illustrating an embodiment in which a strong pressure-sensitive adhesive layer is provided in the probe hole.
[FIG. 18] FIG. 18 shows a plan view of the wearable biosensor in a modified example of the one embodiment (embodiment in which probe and connecter are integrated).
[FIG. 19] FIG. 19 shows a cross sectional view along line A-A of the wearable biosensor shown in FIG. 18.
[FIG. 20] FIG. 20 shows a cross-sectional view of a modified example, in which the probe is larger than the connecter.
[FIG. 21] FIG. 21 shows an enlarged perspective view of the probe and connecter shown in FIG. 20.
[FIG. 22] FIG. 22A and FIG. 22B are cross sectional views of the wearable biosensor in a modified example of the one embodiment, FIG. 22A illustrating an embodiment in which a protection layer including a protection substrate and a second pressure-sensitive adhesive layer is provided, and FIG. 22B illustrating an embodiment in which a protection layer composed only of the protection substrate is provided.

### Description of the embodiments

### <One embodiment>

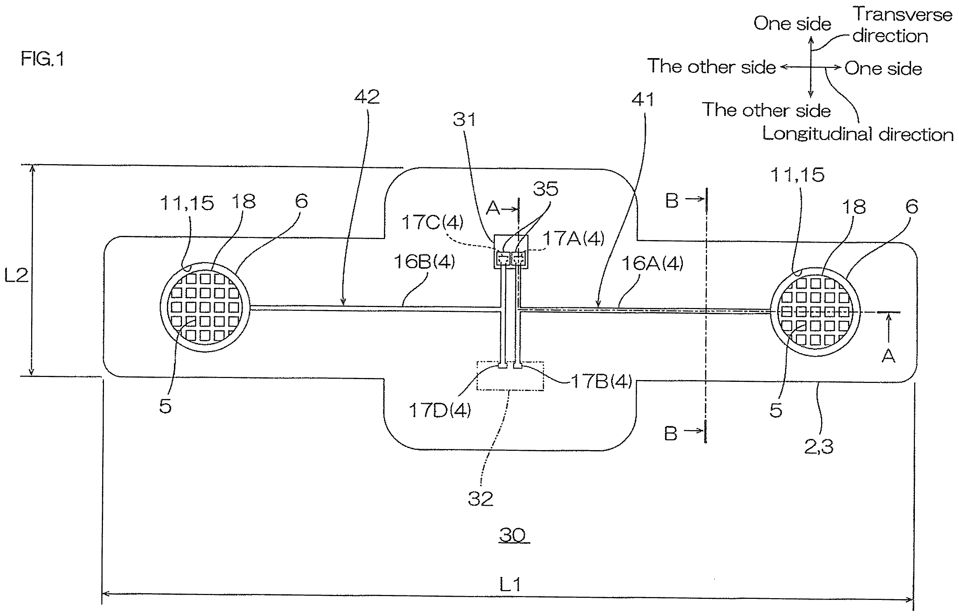

A wearable biosensor 30 as one embodiment of the biosensor of the present invention is described with reference to FIG. 1 to FIG. 6C.

In FIG. 1, left-right direction on the sheet is longitudinal direction (first direction) of the wearable biosensor 30. Right side on the sheet is longitudinal one side (one side in first direction), left side on the sheet is longitudinal other side (the other side in first direction).

In FIG. 1, up-down direction on the sheet is transverse direction (direction orthogonal to longitudinal direction, width direction, second direction orthogonal to first direction) of the wearable biosensor 30. Upper side on the sheet is one side in transverse direction (one side in width direction, one side in second direction), and lower side on the sheet is the other side in transverse direction (the other side in width direction, the other side in second direction).

In FIG. 1, paper thickness direction on the sheet is up-down direction (thickness direction, third direction orthogonal to first direction and second direction) of the wearable biosensor 30. Near side on the sheet is upper side (one side in thickness direction, one side in third direction), and far side on the sheet is lower side (the other side in thickness direction, the other side in third direction).

The directions are in accordance with the direction arrows described in the figures.

These definitions of the directions are not intended to limit the orientations of the wearable biosensor 30 at the time of production and use.

As shown in FIG. 1 to FIG. 2B, the wearable biosensor 30 has a substantially flat plate shape extending in longitudinal direction. The wearable biosensor 30 includes a pressure-sensitive adhesive layer 2, a substrate layer 3 disposed on an adhesive upper face as an example of the upper face of the pressure-sensitive adhesive layer 2, a wire layer 4 disposed on the substrate layer 3, a probe 5 disposed on an adhesive lower face 9 as an example of the lower face of the pressure-sensitive adhesive layer 2, a connecter 6 that electrically connects the wire layer 4 with the probe 5, and an electronic component 31 electrically connected with the wire layer 4.

The pressure-sensitive adhesive layer 2 forms the lower face of the wearable biosensor 30. The pressure-sensitive adhesive layer 2 is a layer that gives pressure-sensitive adhesiveness to the lower face of the wearable biosensor 30 for attaching the lower face of the wearable biosensor 30 to the surface of the living body (skin 33 shown by phantom line, etc.). The pressure-sensitive adhesive layer 2 forms the outline shape of the wearable biosensor 30. The pressure-sensitive adhesive layer 2 has a flat plate shape extending in longitudinal direction. To be specific, the pressure-sensitive adhesive layer 2 has, for example, a band shape extending in longitudinal direction, with a longitudinal center portion bulging toward transverse both outsides. In the pressure-sensitive adhesive layer 2, both end edges in transverse direction of the longitudinal center portion are positioned at transverse both outsides relative to the both end edges in transverse direction of other than the longitudinal center portion.

The pressure-sensitive adhesive layer 2 has an adhesive upper face 8 and an adhesive lower face 9.

The adhesive upper face 8 has a flat face.

The adhesive lower face 9 is disposed to face each other at a lower side of the adhesive upper face 8 in spaced apart relation.

The pressure-sensitive adhesive layer 2 has two adhesion openings 11 at longitudinal both ends thereof. Each of the two adhesion openings 11 has a substantially ring shape in plan view. The adhesion opening 11 penetrates the pressure-sensitive adhesive layer 2 in thickness direction. The adhesion opening 11 is filled with the connecter 6.

The adhesive lower face 9 inside the adhesion opening 11 has adhesion grooves 10 in correspondence with the probe 5 (described later). The adhesion groove 10 is opened toward the lower side.

The material of the pressure-sensitive adhesive layer 2 is not particularly limited as long as it has, for example, pressure-sensitive adhesiveness, and for example, a biocompatible material is used. For such a material, acrylic pressure-sensitive adhesives and silicone pressure-sensitive adhesives are used. Preferably, acrylic pressure-sensitive adhesives are used. For the acrylic pressure-sensitive adhesive, the acrylic polymer described in Japanese Unexamined Patent Publication No. 2003-342541 is used.

When the pressure-sensitive adhesive layer 2 is subjected to a skin peel test, the skin peel area percentage is, for example, 50% or less, preferably 30% or less, more preferably 15% or less, and for example, 0% or more. When the skin peel area percentage is the above-described upper limit or less, even if the pressure-sensitive adhesive layer 2 is attached to the living body, burden on the living body can be suppressed. That is, the material of the pressure-sensitive adhesive layer 2 can have excellent biocompatibility. The skin peel test is conducted based on the method described in Japanese Unexamined Patent Publication No. 2004-83425.

The pressure-sensitive adhesive layer 2 has permeability of, for example, 300 (g/m²/day) or more, preferably 600 (g/m²/day) or more, more preferably 1000 (g/m²/day) or more. When the pressure-sensitive adhesive layer 2 has a permeability of the above-described lower limit or more, even if the pressure-sensitive adhesive layer 2 is attached to a living body, burden on the living body can be suppressed. That is, the material of the pressure-sensitive adhesive layer 2 can have excellent biocompatibility.

The material of the pressure-sensitive adhesive layer 2 is biocompatible when at least one of the following requirements is satisfied: (1) skin peel area percentage is 50% or less in skin peel test, and (2) permeability is 300(g/m²/day) or more, but preferably, the material of the pressure-sensitive adhesive layer 2 satisfy both of (1) and (2).

The pressure-sensitive adhesive layer 2 has a thickness T2 of the distance between the adhesive upper face 8 and the adhesive lower face 9 in the region other than the adhesion groove 10 of, for example, 10 µm or more, preferably 20 µm or more, and for example, 95 µm or less, preferably 70 µm or less, more preferably 50 µm or less.

When the pressure-sensitive adhesive layer 2 has a thickness T2 of the above-described upper limit or less, the total thickness T1 (described later) of the pressure-sensitive adhesive layer 2 and the substrate layer 3 can be set to below the upper limit, and therefore, the wearable biosensor 30 can be thin, particularly the region other than the electronic component 31 in the wearable biosensor 30 can be thin.

The size of the pressure-sensitive adhesive layer 2 in plan view is suitably set in accordance with the skin 33 (described later) to which the wearable biosensor 30 is attached. The pressure-sensitive adhesive layer 2 has a longitudinal direction length L1 of, for example, 30 mm or more, preferably 50 mm or more, and for example, 1000 mm or less, preferably 200 mm or less. The pressure-sensitive adhesive layer 2 has a transverse direction length L2 of, for example, 5 mm or more, preferably 10 mm or more, and for example, 300 mm or less, preferably 100 mm or less.

The pressure-sensitive adhesive layer 2 has a flat area of, for example, 150 mm² or more, preferably 500 mm² or more, more preferably 900 mm² or more, and for example, 300000 mm² or less, preferably 20000 mm² or less, more preferably 10000 mm² or less.

The substrate layer 3 forms the upper face of the wearable biosensor 30 along with the electronic component 31 to be described later. The substrate layer 3 forms the outline shape of the wearable biosensor 30 along with the pressure-sensitive adhesive layer 2. The shape in plan view of the substrate layer 3 is the same as the shape in plan view of the pressure-sensitive adhesive layer 2. The substrate layer 3 is disposed on the entire upper face of the pressure-sensitive adhesive layer 2 (but excluding the region where connecter 6 is provided). The substrate layer 3 is a support layer supporting the pressure-sensitive adhesive layer 2. The substrate layer 3 has a flat plate shape extending in longitudinal direction. The substrate layer 3 has a substrate lower face 12 and a substrate upper face 13.

The substrate lower face 12 has a flat face. The substrate lower face 12 is in contact with (pressure sensitive adhesion) the adhesive upper face 8 of the pressure-sensitive adhesive layer 2.

The substrate upper face 13 is disposed to face each other at the upper side of the substrate lower face 12 in spaced apart relation. The substrate upper face 13 has a substrate groove 14 in correspondence with the wire layer 4. The substrate groove 14 has the same pattern as that of the wire layer 4 in plan view. The substrate groove 14 is opened toward the upper side.

The substrate layer 3 has a substrate opening 15 in correspondence with the adhesion opening 11. The substrate opening 15 communicates with the adhesion opening 11 in thickness direction. The substrate opening 15 has a substantially ring shape in plan view with the same shape and the same size as those of the adhesion opening 11.

The material of the substrate layer 3 has, for example, a stretching property. The material of the substrate layer 3 has, for example, an insulating layer. For such a material, for example, resin is used. Examples of the resin include thermoplastic resin such as polyurethane resin, silicone resin, acrylic resin, polystyrene resin, vinyl chloride resin, and polyester resin.

For the material of the substrate layer 3, in view of ensuring excellent stretching property even more, preferably, polyurethane resin is used.

The substrate layer 3 has an elongation at break of, for example, 100% or more, preferably 200% or more, more preferably 300% or more, and for example, 2000% or less. When the elongation at break is the above-described lower limit or more, the material of the substrate layer 3 can have excellent stretching property. The elongation at break is measured in accordance with JIS K 7127 (1999) with a tensile speed of 5 mm/min and a test piece type 2.

The tensile strength of the substrate layer 3 at 20°C (100 mm between the chucks, tensile speed 300 mm/min, strength at break) is, for example, 0.1N/20 mm or more, preferably 1N/20 mm or more, and for example, 20N/20 mm or less. The tensile strength is measured in accordance with JIS K 7127(1999).

Furthermore, the tensile storage modulus E' of the substrate layer 3 at 20°C is, for example, 2,000 MPa or less, preferably 1,000 MPa or less, more preferably 100 MPa or less, more preferably 50 MPa or less, particularly preferably 20 MPa or less, and for example, 0.1MPa or more. When the tensile storage modulus E' of the substrate layer 3 is the above-described upper limit or less, the material of the substrate layer 3 can have excellent stretching property. The tensile storage modulus E' of the substrate layer 3 at 20°C can be determined by measuring the dynamic viscoelasticity of the substrate layer 3 under conditions of a frequency of 1Hz and a temperature increase speed of 10°C/min.

The material of the substrate layer 3 has stretching property when at least one, preferably two or more, more preferably all of three of the following requirements are satisfied: (3) elongation at break of 100% or more, (4) tensile strength of 20N/20 mm or less, (5) tensile storage modulus E' of 2,000 MPa or less.

The substrate layer 3 has a thickness T3 as a distance between the substrate lower face 12 and the substrate upper face 13 in a region other than the substrate groove 14 of, for example, 1 µm or more, preferably 5 µm or more, and for example, 95 µm or less, preferably 50 µm or less, more preferably 10 µm or less.

The total thickness T1 of the pressure-sensitive adhesive layer 2 and the substrate layer 3, that is, a total T1 (T2+ T3) of the thickness T2 of the pressure-sensitive adhesive layer 2 and the thickness T3 of the substrate layer 3 is 1 µm or more, preferably 10 µm or more, and less than 100 um, preferably 70 µm or less, more preferably 50 µm or less. The total thickness T1 of the pressure-sensitive adhesive layer 2 and the substrate layer 3 is the distance between the adhesive lower face 9 of the pressure-sensitive adhesive layer 2 and the substrate upper face 13 of the substrate layer 3, and the thickness of the wire layer 4 and the probe 5 to be described later is not included.

When the total thickness T1 of the pressure-sensitive adhesive layer 2 and the substrate layer 3 is more than the above-described upper limit, discomfort in wearing of the wearable biosensor 30 for the living body cannot be decreased, and the production costs of the wearable biosensor 30 cannot be decreased, and therefore, in view of costs, it is difficult to make the wearable biosensor 30 disposable. In contrast, when the total thickness T1 of the pressure-sensitive adhesive layer 2 and the substrate layer 3 is below the above-described upper limit, discomfort in wearing of the wearable biosensor 30 for a living body can be decreased, and the production costs can be decreased, and therefore, the wearable biosensor 30 can be made a disposable type.

Meanwhile, when the total thickness T1 of the pressure-sensitive adhesive layer 2 and the substrate layer 3 is more than the above-described lower limit, handleability of the wearable biosensor 30 can be improved.

The wire layer 4 is embedded in a substrate groove 14. To be specific, the wire layer 4 is embedded in the upper portion of the substrate layer 3 so as to be exposed from the substrate upper face 13 of the substrate layer 3. The wire layer 4 has an upper face and a lower face disposed in spaced apart relation from each other, and side faces connecting their peripheral end edges. The entire lower face and the entire side face are in contact with the substrate layer 3. The upper face is exposed from the substrate upper face 13 (excluding substrate groove 14). The upper face of the wire layer 4 forms the upper face of the wearable biosensor 30 along with the substrate upper face 13 and the electronic component 31.

The wire layer 4 has a wire pattern connecting the connecter 6, an electronic component 31 (described later), and a battery 32 (described later). To be specific, the wire layer 4 independently includes a first wire pattern 41 and a second wire pattern 42.

The first wire pattern 41 is disposed at longitudinal one side of the substrate layer 3. The first wire pattern 41 includes a first wire 16A, and a first terminal 17A and a second terminal 17B continuous therefrom.

The first wire pattern 41 has a substantially letter T-shape in plan view. To be specific, the first wire pattern 41 extends from the longitudinal one end portion (the connecter 6 positioned at) of the substrate layer 3 toward longitudinal other side, splits at the longitudinal center portion of the substrate layer 3, and extends toward transverse both outsides.

The first terminal 17A and the second terminal 17B each is disposed at transverse both end portions in longitudinal center portion of the substrate layer 3. The first terminal 17A and the second terminal 17B each has a substantially rectangular shape in plan view (land shape). The first terminal 17A and the second terminal 17B each is continuous with both end portions of the first wire 16A extending in transverse both outsides at a longitudinal center portion of the substrate layer 3.

The second wire pattern 42 is provided in spaced apart relation at longitudinal other side of the first wire pattern 41. The second wire pattern 42 includes a second wire 16B and a third terminal 17C and a fourth terminal 17D continuous therefrom.

The second wire pattern 42 has a substantially letter T-shape in plan view. To be specific, the second wire pattern 42 extends from (the connecter 6 positioned at) the longitudinal other end portion of the substrate layer 3 toward longitudinal one side, splits at the longitudinal center portion of the substrate layer 3, and extends toward transverse both outsides.

The third terminal 17C and the fourth terminal 17D each is disposed at transverse both end portions in longitudinal center portion of the substrate layer 3. The third terminal 17C and the fourth terminal 17D each has a substantially rectangular shape in plan view (land shape). The third terminal 17C and the fourth terminal 17D each is continuous with both end portions of the second wire 16B extending in transverse both outsides at a longitudinal center portion of the substrate layer 3.

For the material of the wire layer 4, for example, conductors such as copper, nickel, gold, and alloys thereof are used. For the material of the wire layer 4, preferably, copper is used.

The wire layer 4 has a thickness T4 of, for example, smaller than the thickness T3 of the substrate layer 3. To be specific, the wire layer 4 has a thickness T4 of, for example, 0.1 µm or more, preferably 1 µm or more, and for example, 100 µm or less, preferably 5 0 µm or less.

The probe 5 is an electrode that allows sensing of electric signals, temperatures, vibrations, sweat, and metabolite from a living body, when the pressure-sensitive adhesive layer 2 is attached to the skin 33 by making contact with the skin 33. The probe 5 is embedded in the pressure-sensitive adhesive layer 2 so as to be exposed from the adhesive lower face 9 of the pressure-sensitive adhesive layer 2. That is, the probe 5 is embedded in the adhesion groove 10 of the pressure-sensitive adhesive layer 2 at the inside of the adhesion opening 11. The probe 5 is disposed at the adhesive lower face 9 forming the adhesion groove 10. That is, the probe 5 is embedded in the lower end portion of the pressure-sensitive adhesive layer 2 at the inside of the adhesion opening 11. The probe 5 has a mesh shape, preferably, a substantially grid shape in plan view (or has a substantially mesh shape). In other words, the probe 5 has holes in spaced apart relation in the surface direction (longitudinal direction and transverse direction). The hole is filled with the pressure-sensitive adhesive layer 2.

The probe 5 has a substantially rectangular shape in cross sectional view extending in a direction orthogonal thereto. The probe 5 has a probe lower face 20, a probe upper face 21 disposed to face the upper side of the probe lower face 20 in spaced apart relation, and side faces connecting peripheral end edges of the probe lower face 20 and the probe upper face 21.

The probe lower face 20 is exposed from the adhesive lower face 9 (excluding adhesion groove 10) of the pressure-sensitive adhesive layer 2. The probe lower face 20 is flush with the adhesive lower face 9. The probe lower face 20 forms the lower face of the wearable biosensor 30 along with the adhesive lower face 9.

The probe upper face 21 and the side face are covered with the pressure-sensitive adhesive layer 2.

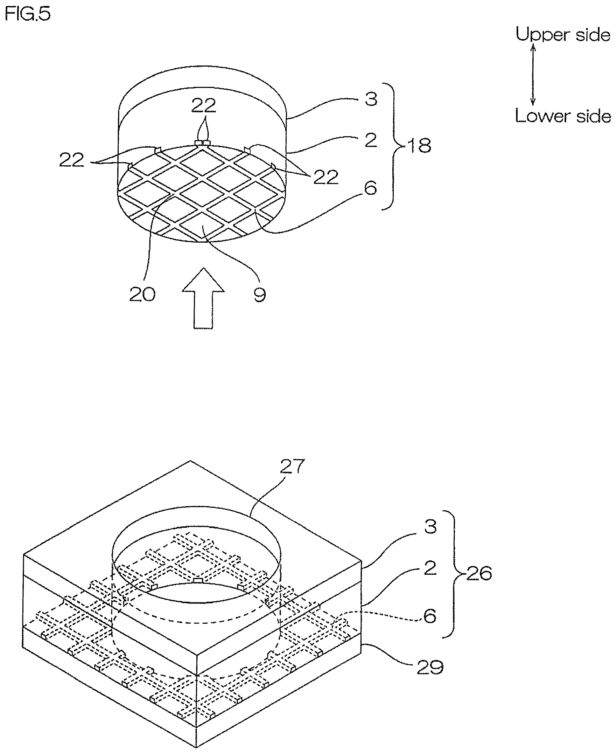

As shown in FIG. 5, of the side faces of the probe 5, the face positioned at the outermost side is an outer side face 22. The outer side face 22 forms a virtual circle passing through the outer side face 22 in plan view.

For the material of the probe 5, those materials given as examples of the wire layer 4 (to be specific, conductor) are used.

The external size of the probe 5 is set so that the virtual circle passing through the outer side face 22 overlaps with the inner periphery defining the adhesion opening 11 in plan view.

The probe 5 has a thickness T5 of, for example, smaller than the thickness T2 of the pressure-sensitive adhesive layer 2. To be specific, the probe 5 has a thickness T5 of, for example, 0.1 µm or more, preferably 1 µm or more, and for example, 100 µm or less, preferably 50 µm or less.

The connecter 6 is provided in correspondence with the substrate opening 15 and the adhesion opening 11, and has the same shape as these. The connecter 6 penetrates (pass through) the substrate layer 3 and the pressure-sensitive adhesive layer 2 in thickness direction (up-down direction), and the substrate opening 15 and the adhesion opening 11 are filled with the connecter 6. The connecter 6 has a no-end shape in plan view along the outer side face 22 of the probe 5. To be specific, the connecter 6 has a substantially cylindrical shape with its axis line extending in thickness direction (along virtual circle passing through the outer side face 22).

The inner side face of the connecter 6 is in contact with the outer side face 22 of the probe 5.

The connecter 6 is allowed to adhere to the pressure-sensitive adhesive layer 2 outside the adhesion opening 11 and the pressure-sensitive adhesive layer 2 inside the adhesion opening 11 by pressure-sensitive adhesion. The connecter 6 is in contact with the substrate layer 3 outside the substrate opening 15 and the substrate layer 3 inside the substrate opening 15.

The upper face of the connecter 6 is flush with the substrate upper face 13. The lower face of the connecter 6 is flush with the adhesive lower face 9.

As shown in FIG. 1, of the two connecters 6, the connecter 6 positioned at longitudinal one side is continuous with, at its upper end portion, longitudinal one end edge of the wire 16A positioned at longitudinal one side. The connecter 6 positioned at longitudinal other side is continuous with, at its upper end portion, longitudinal other end edge of the wire 16B positioned at longitudinal other side.

That is, the connecter 6 is electrically connected with the wire layer 4.

In this manner, the connecter 6 electrically connects the wire layer 4 with the probe 5.

The connecter 6 and the wire layer 4 form a circuit portion 36 that electrically connects the probe 5 with the electronic component 31. That is, the circuit portion 36 includes the wire layer 4 disposed on the substrate upper face 13 of the substrate layer 3, and the connecter 6 passing through the substrate layer 3 and the pressure-sensitive adhesive layer 2. Preferably, the circuit portion 36 is composed only of the wire layer 4 and the connecter 6.

For the material of the connecter 6, metals and electrically conductive resin (including electrical conductive polymer) are used, and preferably, electrically conductive resin is used.

The thickness of the connecter 6 (up-down direction length) is the same as a total thickness T1 of the pressure-sensitive adhesive layer 2 and substrate layer 3. The radial direction length of the connecter 6 (half the value deducting internal diameter from external diameter) is, 1 µm or more, preferably 100 µm or more, and less than 2000 µm, preferably 1000 µm or less, more preferably 500 µm or less.

Examples of the electronic component 31 include an analog front-end, microcomputer, and memory for processing and storing electric signals from a living body obtained by the probe 5, and a communication IC and transmitter for converting electric signals to electro-magnetic waves and wirelessly transmitting them to an external receiver.

To be more specific, when the wearable biosensor 30 is a wearable electrocardiograph, the changes in cardiac potential obtained at the probe 5 is converted to digital data at an analog front-end, and the cardiac potential is stored in the memory. For example, the cardiac potential is stored in the memory with 16 bit, at a data rate of 1kHz. To decrease the memory size, resolving power of data and data rate have to be decreased. After detaching the wearable biosensor 30 after the measurement, the stored data is taken out from the memory and analyzed. The communication IC has functions to send the signals obtained at the probe 5 to outside wirelessly. This function works when connected under normal communication, the wearable biosensor 30 is attached to the skin 33, and when it can be confirmed that data acquisition is normal, and a message that the data acquisition is normal is intermittently sent to outside, to check if the wearable biosensor 30 is working normally.

The electronic component 31 can have some or all of the above-described components. The electronic component 31 is in contact with the substrate upper face 13. The electronic component 31 has a substantially rectangular flat plate shape in cross sectional view. Two terminals 35 are provided at the lower face of the electronic component 31. Two terminals of the electronic component 31 are electrically connected with the first terminal 17A and the third terminal 17C, respectively. The electronic component 31 is harder than, for example, the pressure-sensitive adhesive layer 2 and the substrate layer 3.

The electronic component 31 has a thickness T6 of, for example, 1 µm or more, preferably 10 µm or more, and for example, 1000 µm or less, preferably 500 µm or less.

When the electronic component 31 has a thickness T6 of the above-described upper limit or less, the total thickness T7 of the wearable biosensor 30 can be small. When the electronic component 31 has a thickness T6 of the above-described lower limit or more, handleability and mountability of the electronic component 31 can be improved.

The electronic component 31 has a flat area S, that is, a cross sectional area S when the electronic component 31 is cut along the surface direction, of, for example, 0.001 mm² or more, preferably 0.01 mm² or more, more preferably 0.05 mm² or more, and for example, 10 mm² or less, preferably 2 mm² or less, more preferably 1 mm² or less. When the electronic component 31 has a flat area S of the above-described upper limit or less, discomfort in wearing of the wearable biosensor 30 for a living body can be decreased even more.

When the electronic component 31 has a flat area S of the above-described lower limit or more, handleability and mountability of the electronic component 31 can be improved.

The wearable biosensor 30 has a thickness T7 of a total of a total thickness T1 of the above-described pressure-sensitive adhesive layer 2 and substrate layer 3, and a thickness T6 of the electronic component 31 (T2+ T3+ T6), and for example, it is 2 µm or more, preferably 20 µm or more, and for example, 1000 µm or less, preferably 100 µm or less.

Next, description is given below of the method for producing a wearable biosensor 30.

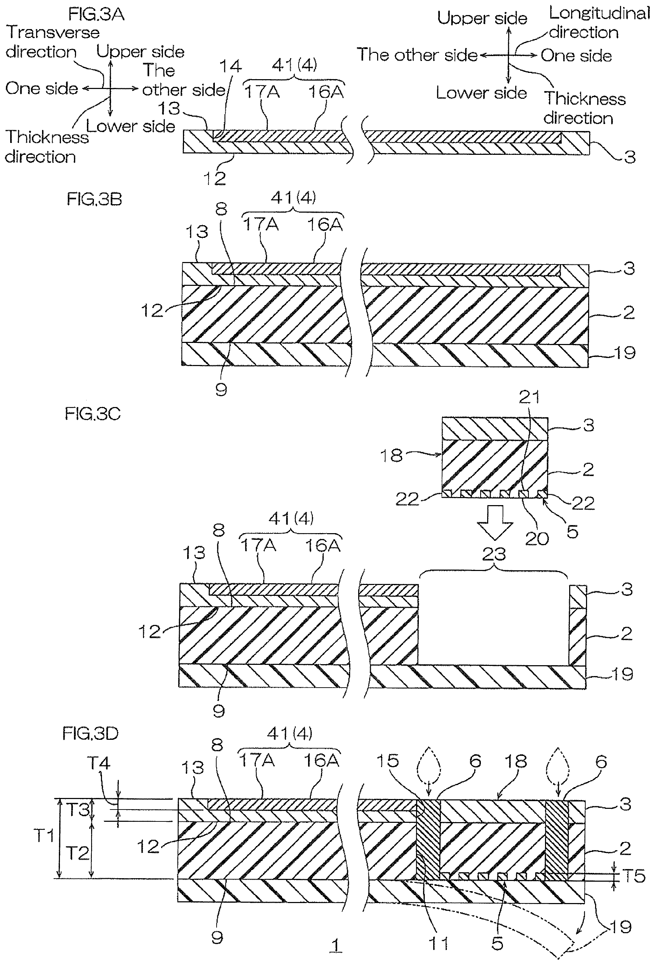

As shown in FIG. 3A, in this method, first, the substrate layer 3 and wire layer 4 are prepared.

For example, the substrate layer 3 and wire layer 4 are prepared so that the wire layer 4 is embedded in the substrate groove 14 by the method described in Japanese Unexamined Patent Publication No. 2017-22236 and Japanese Unexamined Patent Publication No. 2017-22237.

As shown in FIG. 3B, then, the pressure-sensitive adhesive layer 2 is disposed on the substrate lower face 12.

To dispose the pressure-sensitive adhesive layer 2 on the substrate lower face 12, for example, first, an application liquid containing the materials for the pressure-sensitive adhesive layer 2 is prepared, and then the application liquid is applied on the upper face of the first release sheet 19, and thereafter, they are dried by heating. In this manner, the pressure-sensitive adhesive layer 2 is disposed on the upper face of the first release sheet 19. The first release sheet 19 has, for example, a substantially flat plate shape extending in longitudinal direction. For the material of the first release sheet 19, for example, resin such as polyethylene terephthalate is used.

Then, the pressure-sensitive adhesive layer 2 and the substrate layer 3 are bonded by, for example, a laminator. To be specific, the adhesive upper face 8 of the pressure-sensitive adhesive layer 2 is brought into contact with the substrate lower face 12 of the substrate layer 3.

At this point, the substrate layer 3 or the pressure-sensitive adhesive layer 2 has no substrate opening 15 or adhesion opening 11.

As shown in FIG. 3C, then, the opening 23 is formed in the substrate layer 3 and the pressure-sensitive adhesive layer 2.

The opening 23 penetrates the substrate layer 3 and pressure-sensitive adhesive layer 2. The opening 23 is a hole having a generally circular shape in plan view (through opening) defined by an outer peripheral face defining the substrate opening 15 and an outer peripheral face defining the adhesion opening 11. The opening 23 is opened toward the upper side. Meanwhile, the lower end of the opening 23 is closed by the first release sheet 19.

To form the opening 23, the pressure-sensitive adhesive layer 2 and substrate layer 3 are subjected to, for example, punching or half etching.

Then, the probe member 18 is prepared, and inserted into the opening 23.

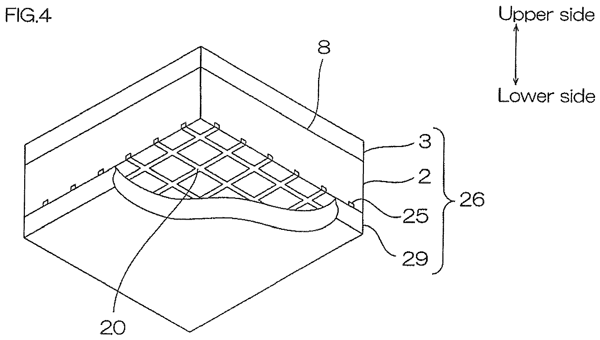

As shown in FIG. 4, to prepare the probe member 18, first, the probe-containing sheet 26 is prepared.

The probe-containing sheet 26 includes a second release sheet 29, a probe pattern 25 formed on the second release sheet 29, a pressure-sensitive adhesive layer 2 formed on the second release sheet 29 and in which the probe pattern 25 is embedded, and a substrate layer 3 disposed on the adhesive upper face 8 of the pressure-sensitive adhesive layer 2.

The second release sheet 29 has the same configuration as that of the above-described first release sheet 19.

The probe pattern 25 has the same pattern as that of the probe 5, and the material of the probe pattern 25 is the same as the material of the probe 5. The probe pattern 25 has a flat area larger than the virtual circle passing through the outer side face 22 of the probe 5.

The pressure-sensitive adhesive layer 2 and substrate layer 3 of the probe-containing sheet 26 has the same configuration as that of the above-described pressure-sensitive adhesive layer 2 and substrate layer 3.

The probe-containing sheet 26 is prepared, for example, by the method described in Japanese Unexamined Patent Publication No. 2017-22236 and Japanese Unexamined Patent Publication No. 2017-22237.

Although not shown, to be specific, after forming a seed layer composed of copper on the upper face of a release layer composed of stainless steel, a photoresist is laminated on the entire upper face of the seed layer. Then, the photoresist is exposed to light and developed, thereby forming the photoresist into a reverse pattern of the probe pattern 25. Then, after the probe pattern 25 is formed on the upper face of the seed layer by electrolytic plating, the photoresist is removed. Thereafter, an application liquid containing the material of the pressure-sensitive adhesive layer 2 is applied to cover the probe pattern 25, and cured to form the pressure-sensitive adhesive layer 2. Then, the substrate layer 3 is bonded to the upper face of the pressure-sensitive adhesive layer 2 by, for example, a laminator. Then, the release layer is removed from the lower face of the seed layer, and then the seed layer is removed. Thereafter, as necessary, the second release sheet 29 is bonded to the lower face of the pressure-sensitive adhesive layer 2. The second release sheet 29 has the same configuration as that of the above-described first release sheet 19.

In this manner, the probe-containing sheet 26 is prepared.

As shown in FIG. 5, then, a cutting line 27 is formed on the probe pattern 25, pressure-sensitive adhesive layer 2, and substrate layer 3 into a generally circular shape in plan view. The cutting line 27 is formed, for example, by punching. The cutting line 27 divides the probe pattern 25, pressure-sensitive adhesive layer 2, and substrate layer 3 into inner portions and outer portions, but the cutting line 27 is not formed on the second release sheet 29. The size of the cutting line 27 is the same as the internal diameter of the adhesion opening 11 and substrate opening 15. That is, the cutting line 27 coincides with the virtual circle passing through the outer side face 22.

By forming the cutting line 27, the probe member 18 is formed.

In the probe member 18, the outer side face 22 of the probe 5 is flush with the outer side face of the pressure-sensitive adhesive layer 2. In the probe member 18, the outer side face 22 is exposed to the outside in radial direction from the outer side face of the pressure-sensitive adhesive layer 2.

Then, as shown in the arrow in FIG. 5, the probe member 18 is pulled out from the second release sheet 29. To be specific, the adhesive lower face 9 and probe lower face 20 of the probe member 18 are released from the second release sheet 29.

Thereafter, as shown by the arrow in FIG. 3C, the probe member 18 is inserted in the opening 23.

At this time, a gap is created between the pressure-sensitive adhesive layer 2, substrate layer 3, and probe 5 of the probe member 18, and the pressure-sensitive adhesive layer 2 and substrate layer 3 surrounding the opening 23. That is, the probe member 18 is inserted into the opening 23 so as to form the substrate opening 15 and the adhesion opening 11.

Thereafter, as shown in FIG. 3D, the connecter 6 is provided in the substrate opening 15 and the adhesion opening 11.

When the material of the connecter 6 is an electrically conductive resin composition, the electrical conductive resin composition is injected (or applied) to the substrate opening 15 and the adhesion opening 11. Thereafter, as necessary, the electrical conductive resin composition is heated.

In this manner, the biosensor laminate 1 including the first release sheet 19, pressure-sensitive adhesive layer 2, substrate layer 3, wire layer 4, probe 5, and connecter 6 is produced. The biosensor laminate 1 is distributed singly, and is an industrially applicable device. To be specific, the biosensor laminate 1 can be distributed singly, separately from the electronic component 31 and battery 32 (ref: phantom line in FIG. 1) to be described later. That is, the biosensor laminate 1 is not mounted with the electronic component 31 or battery 32, and is a component for producing a wearable biosensor 30.

As shown in FIG. 1, thereafter, the two terminals 35 of the electronic components 31 are electrically connected with the first terminal 17A and the third terminal 17C. At this time, the lower face of the electronic component 31 is allowed to contact the substrate upper face 13.

In this manner, the wearable biosensor 30 is produced.

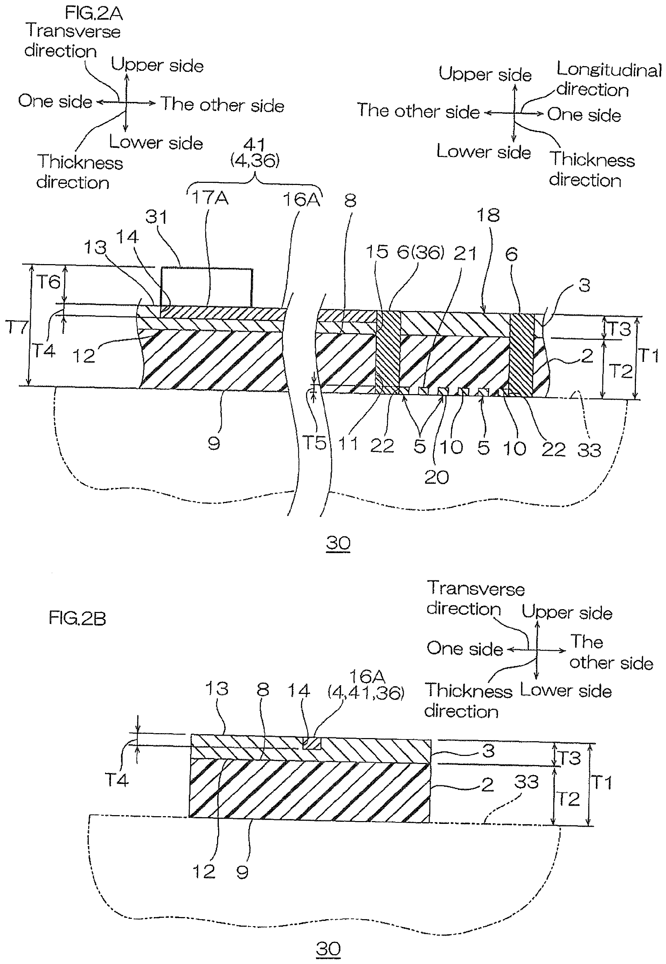

The wearable biosensor 30 includes a pressure-sensitive adhesive layer 2, a substrate layer 3, a wire layer 4, a probe 5, a connecter 6, an electronic component 31, and a first release sheet 19, and preferably, is composed only of these. As shown in FIG. 2A, the wearable biosensor 30 may be composed only of the pressure-sensitive adhesive layer 2, substrate layer 3, wire layer 4, probe 5, connecter 6, and electronic component 31 without including the first release sheet 19.

Next, description is given below of a method of using the wearable biosensor 30.

To use the wearable biosensor 30, first, the battery 32 is mounted on the wearable biosensor 30.

The battery 32 has a substantially flat plate (box) shape extending in surface direction. The battery 32 has two terminals (not shown) provided at its lower face. The battery 32 has a thickness of, for example, 1 µm or more, preferably 10 µm or more, and for example, 1000 µm or less, preferably 100 µm or less.

To allow the battery 32 to be mounted on the wearable biosensor 30, the two terminals (not shown) of the battery 32 are electrically connected with the second terminal 17B and fourth terminal 17D. At that time, the lower face of the battery 32 is allowed to contact the substrate upper face 13.

Then, the first release sheet 19 (ref: arrow and phantom line of FIG. 3D) is released from the pressure-sensitive adhesive layer 2 and probe 5.

As shown in the phantom line in FIG. 2A, then, the adhesive lower face 9 of the pressure-sensitive adhesive layer 2 is allowed to contact, for example, a skin 33 of a human body. To be specific, the pressure-sensitive adhesive layer 2 is allowed to pressure-sensitively adhere to a surface of the skin 33.

Then, the probe lower face 20 of the probe 5 makes contact with the surface of the skin 33, by allowing the adhesive lower face 9 to pressure-sensitively adhere (attaching) to the skin 33.

Then, the probe 5 senses electric signals from the living body, and the electric signals sensed at the probe 5 are inputted to the electronic component 31 through the connecter 6 and wire layer 4. The electronic component 31 processes the electric signal based on the electric power supplied from the battery 32, and store that information. Furthermore, as necessary, the electric signals are converted to electro-magnetic waves, and they are wirelessly transmitted to an external receiver.

Examples of the wearable biosensor 30 include devices that can sense electric signals of a living body and monitor conditions of a living body, and to be specific, a wearable electrocardiograph, wearable electroencephalograph, wearable sphygmomanometer, wearable pulse meter, wearable electromyograph, wearable thermometer, and wearable accelerometer. These devices can be individual devices, or can be a device including the plurality of these devices.

The wearable biosensor 30 is preferably used as a wearable electrocardiograph. In the wearable electrocardiograph, the probe 5 senses cardiac action potential as electric signals.

The living body includes a human body and a living thing other than the human body, but preferably, the living body is a human body.

With the wearable biosensor 30, the total thickness T1 of the pressure-sensitive adhesive layer 2 and the substrate layer 3 is small, i.e., 1 µm or more and less than 100 µm, and therefore even when the wearable biosensor 30 is attached to the skin 33, discomfort in wearing can be sufficiently reduced for a living body, in particular, a human body.

With the wearable biosensor 30, the total thickness T1 of the pressure-sensitive adhesive layer 2 and substrate layer 3 is small, and the electronic component 31 is small, and therefore production costs can be decreased, and therefore, the wearable biosensor 30 can be made disposable.

Meanwhile, the disposed wearable biosensor 30 is collected thereafter, as necessary, and for example, the electronic component 31, and also the battery 32 can be taken out to be recycled.

When the pressure-sensitive adhesive layer 2 has a thickness T2 of the above-described upper limit or less, the total thickness T1 of the pressure-sensitive adhesive layer 2 and substrate layer 3 can be set to be below the upper limit, and the wearable biosensor 30, in particular the region other than the electronic component 31 in the wearable biosensor 30 can be made smaller. The thickness T2 of the pressure-sensitive adhesive layer 2 can give sufficient pressure-sensitive adhesiveness to the skin 33 of the wearable biosensor 30.

When the electronic component 31 has a thickness T6 of the above-described upper limit or less, the total thickness T7 of the wearable biosensor 30 can be made small. When the electronic component 31 has a thickness T6 of the above-described lower limit or more, handleability and mountability of the electronic component 31 can be improved.

When the electronic component 31 has a flat area S of the above-described upper limit or less, discomfort in wearing of the wearable biosensor 30 for a living body can be decreased even more. When the electronic component 31 has a flat area S of the above-described lower limit or more, handleability and mountability of the electronic component 31 can be improved.

### <Modified example>

In the modified examples below, the members and steps corresponding to those described in the embodiment above are designated by the same reference numerals, and detailed descriptions thereof are omitted. These modified examples can be suitably combined. Furthermore, the modified examples have the same operations and effects as those in the embodiment unless otherwise noted.

As shown in FIGs. 1 and 5, in the embodiment, the line passing through the outer side face 22 is circular, but the shape is not particularly limited, and for example, although not shown, it can be rectangular.

As shown in FIG. 2A, in the embodiment, the entire side face (excluding outer side face 22) of the probe 5 is in contact with the pressure-sensitive adhesive layer 2, and the probe lower face 20 of the probe 5 is flush with the adhesive lower face 9 of the pressure-sensitive adhesive layer 2.

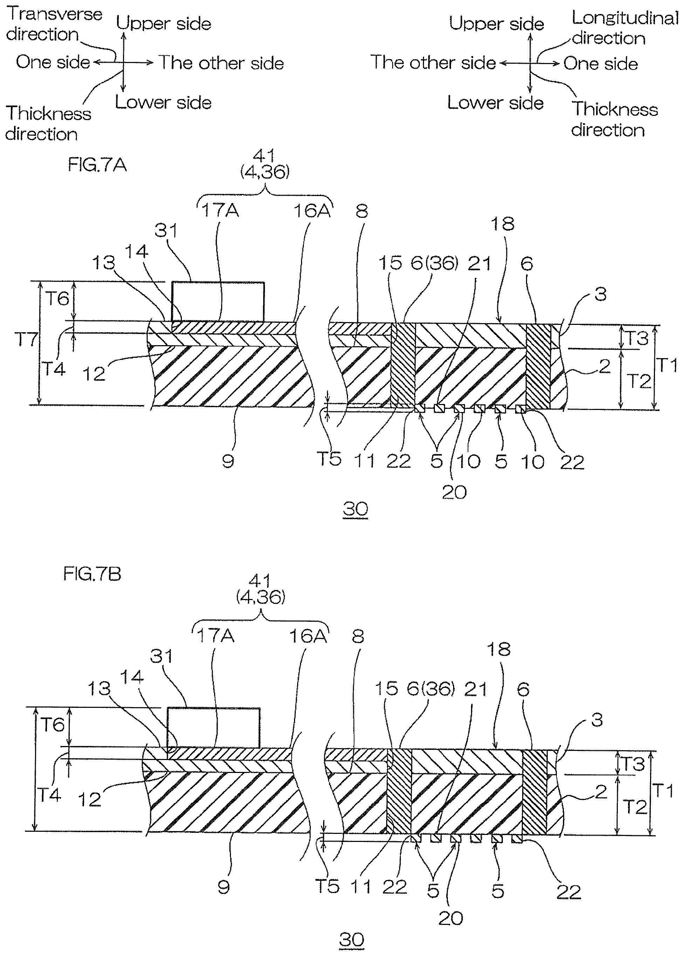

Meanwhile, as shown in FIG. 7A, the upper portion of the side face (excluding outer side face 22) of the probe 5 can contact the pressure-sensitive adhesive layer 2, and the lower portion thereof can be exposed from the adhesive lower face 9. The probe lower face 20 is positioned at the lower side of the adhesive lower face 9. That is, only the upper portion of the probe 5 is embedded in the pressure-sensitive adhesive layer 2, and the lower portion of the probe 5 is projected downward from the adhesive lower face 9.

Furthermore, as shown in FIG. 7B, the entire probe 5 can be projected downward from the adhesive lower face 9. In this case, the adhesive lower face 9 has no adhesion groove 10, and has a flat face.

As shown in FIG. 2B, in the embodiment, the entire side face of the wire layer 4 is in contact with the substrate layer 3.

Meanwhile, as shown in FIG. 8A, in the modified example, the lower portion of the side face of the wire layer 4 is in contact with the substrate layer 3, and the upper portion of the side face of the wire layer 4 is exposed from the substrate upper face 13 of the substrate layer 3. That is, the upper portion of the wire layer 4 is projected from the substrate upper face 13 of the substrate layer 3, and the lower portion of the wire layer 4 is embedded in the substrate layer 3.

As shown in FIG. 8B, the entire side face of the wire layer 4 can be exposed. The substrate upper face 13 has no substrate groove 14, and has a flat face. The lower face of the wire layer 4 is placed on and in contact with the substrate upper face 13.

As shown in FIG. 8C, the wire layer 4 can be embedded entirely in the substrate layer 3. That is, the wire layer 4 is embedded in the substrate layer 3. All of the upper face, lower face, and side face of the wire layer 4 are covered with the substrate layer 3. The wire layer 4 is positioned between the substrate upper face 13 and substrate lower face 12 of the substrate layer 3.

As shown in FIG. 8D, the wire layer 4 is embedded in the substrate layer 3 so as to be exposed from the substrate lower face 12. The lower face of the wire layer 4 is flush with the substrate lower face 12, and in contact with the adhesive upper face 8. In this case, although not shown, the connecter 6 does not pass through the substrate layer 3, but pass through only the pressure-sensitive adhesive layer 2. That is, the connecter 6 is only inserted in the adhesion opening 11.

As shown in FIG. 8E, the wire layer 4 can be provided on both of the pressure-sensitive adhesive layer 2 and the substrate layer 3. To be specific, the upper portion of the wire layer 4 is embedded in the substrate layer 3, and the lower portion of the wire layer 4 is embedded in the pressure-sensitive adhesive layer 2.

As shown in FIG. 8F, the wire layer 4 can be embedded only in the pressure-sensitive adhesive layer 2. The wire layer 4 is exposed from the adhesive upper face 8 of the pressure-sensitive adhesive layer 2.

As shown in FIG. 8G, the wire layer 4 is entirely embedded, without being exposed from any of the adhesive upper face 8 and adhesive lower face 9 of the pressure-sensitive adhesive layer 2.

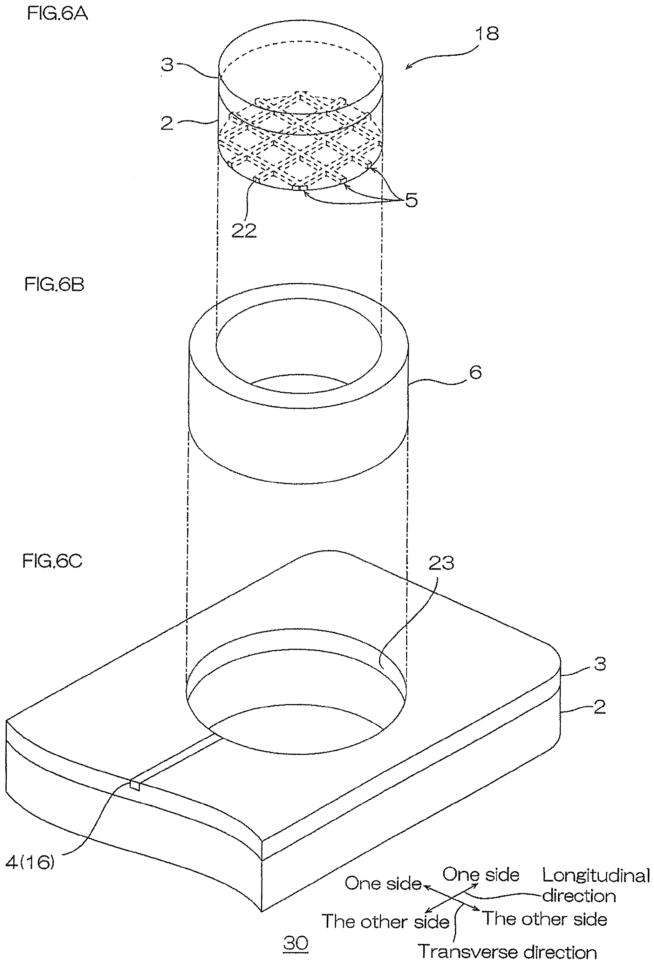

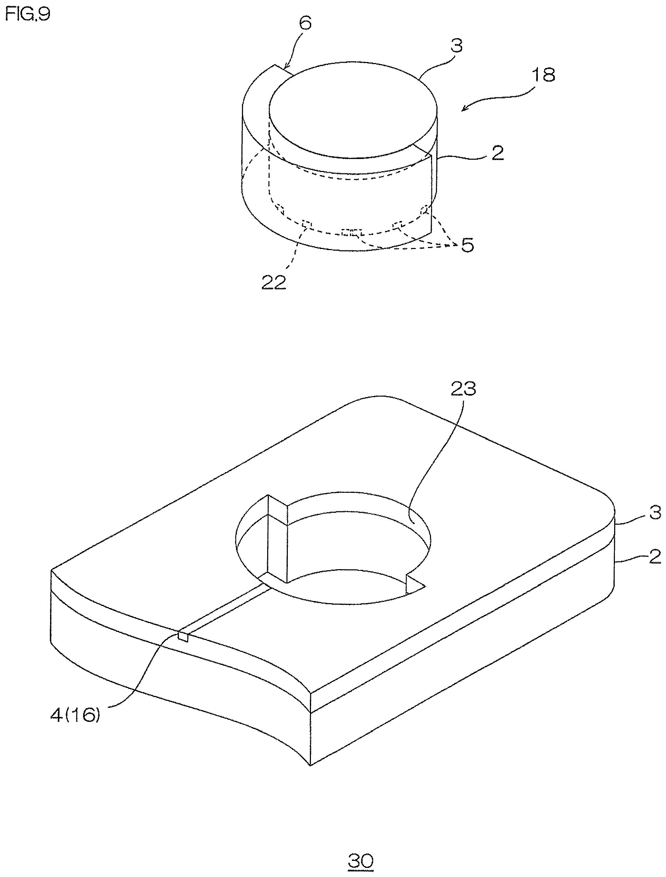

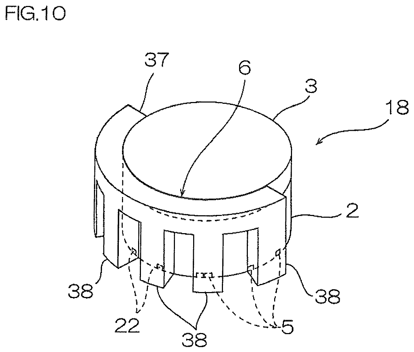

As shown in FIG. 1 and FIG. 6B, in the embodiment, the connecter 6 has a no-end shape in plan view, but for example, as shown in FIGs. 9 and 10, it can have a with-end shape in plan view.

As shown in FIG. 9, the connecter 6 has a substantially half ring shape in plan view (or substantially half-arc shape).

As shown in FIG. 10, the connecter 6 includes an upper end portion 37 having a substantially half ring shape in plan view, and a plurality of first strips 38 continuous to the upper end portion 37.

The first strip 38 extends downward from the lower end edge of the upper end portion 37. The lower end portion of the first strip 38 is in contact with the outer side face 22. The plurality of first strips 38 are disposed in spaced apart relation along the virtual half-circle along the upper end portion 37.

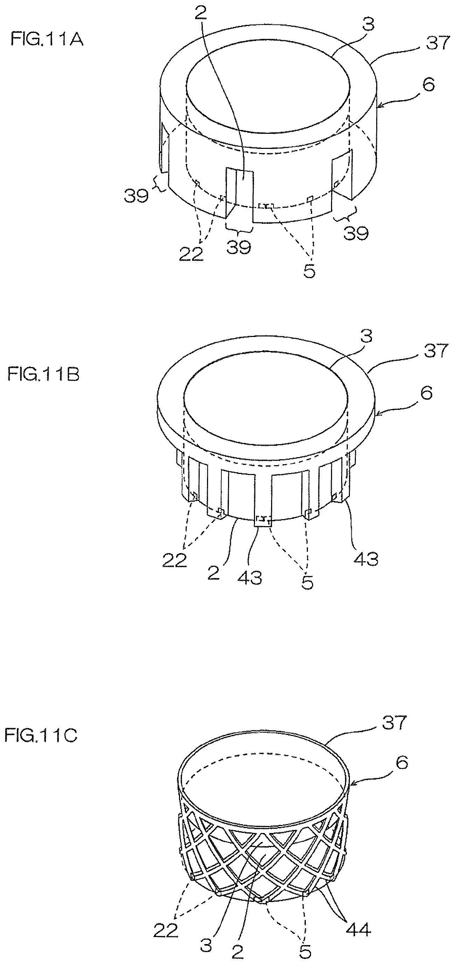

As shown in FIG. 11A to FIG. 11C, the upper end portion 37 of the connecter 6 can have a no-end shape in plan view, and the portion positioned at the lower side than the upper end portion 37 can have a with-end shape in bottom view (or cross sectional view along the surface direction (cross sectional view in plan view)).

As shown in FIG. 11A to FIG. 11C, the upper end portion 37 in the connecter 6 has a substantially ring shape in plan view.

As shown in FIG. 11A, the connecter 6 has a slit 39 formed at the lower side of the upper end portion 37. The slit 39 is provided in a plural number in spaced apart relation along the virtual circle along the upper end portion 37. That is, the connecter 6 has a substantially cylindrical shape with a plurality of slit 39 formed.

As shown in FIG. 11B, the connecter 6 has an upper end portion 37, and a second strip 43 descending from the lower end edge thereof. The second strip 43 is provided in a plural number in spaced apart relation along the virtual circle along the upper end portion 37. The lower end portion of the plurality of second strips 43 are in contact with the outer side face 22.

As shown in FIG. 11C, the connecter 6 integrally includes the upper end portion 37, and a mesh portion 44 positioned at the lower side of the upper end portion 37. The upper end portion of the mesh portion 44 is continuous with the lower end edge of the upper end portion 37. The lower end portion of the mesh portion 44 is in contact with the outer side face 22.

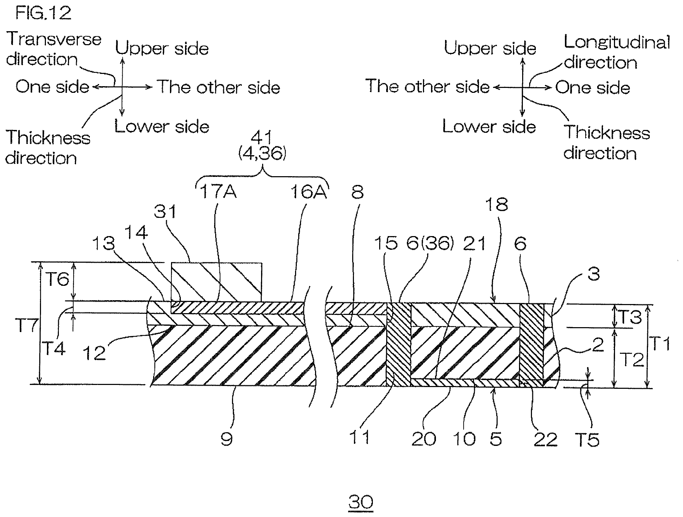

As shown in FIG. 12, the probe 5 can have no hole, and can have a substantially flat plate shape (to be specific, substantially disc shape) extending in surface direction. The outer peripheral face of the probe 5 is in contact with the inner periphery of the lower end portion of the connecter 6.

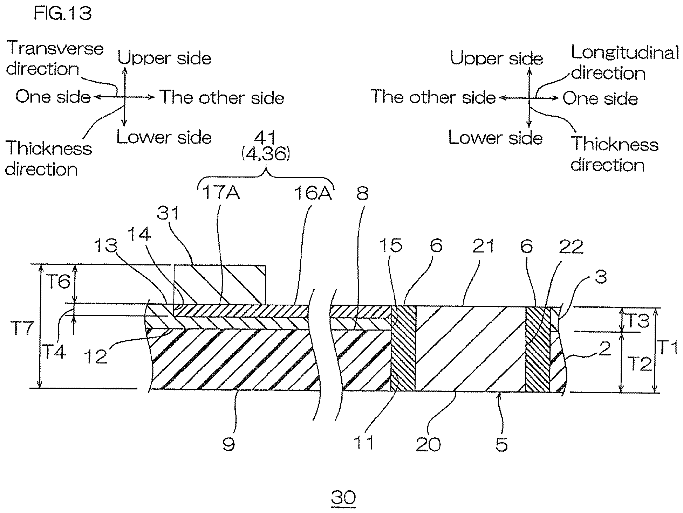

As shown in FIG. 13, the probe 5 can be a substantially pillar shape (to be specific, substantially cylindrical) passing through the pressure-sensitive adhesive layer 2 and substrate layer 3. The probe upper face 21 is exposed from the substrate upper face 13 of the substrate layer 3 and the upper face of the connecter 6. The entire outer peripheral face of the probe 5 is in contact with the entire inner periphery of the connecter 6.

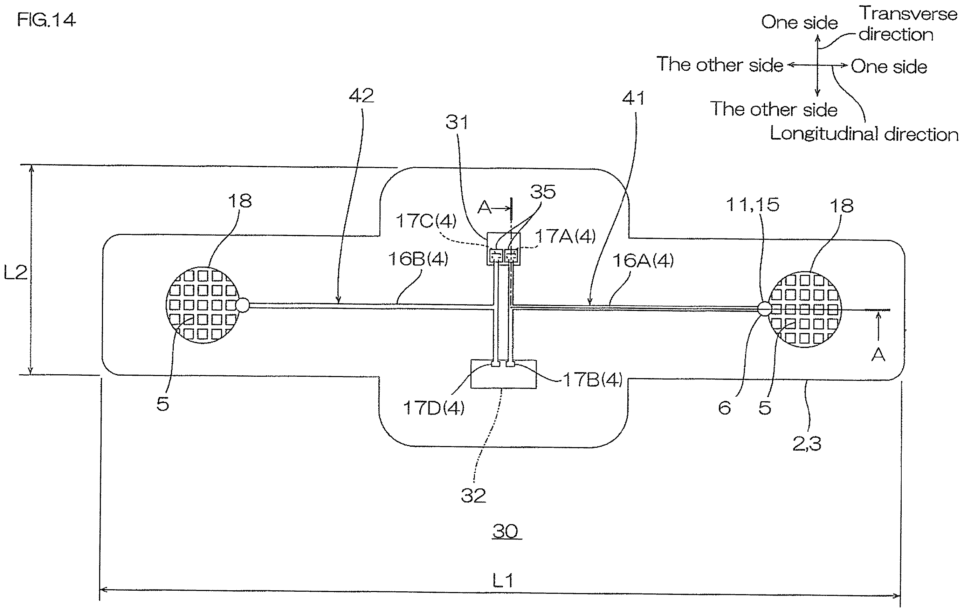

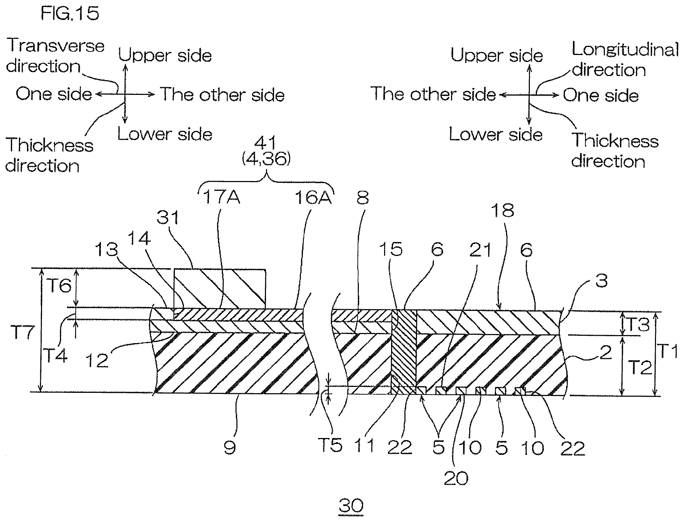

As shown in FIGs. 14 and 15, the connecter 6 can have a substantially rod (round rod) (needle) shape with its axis line extending in thickness direction.

The connecter 6 makes contact with the probe 5 in points.

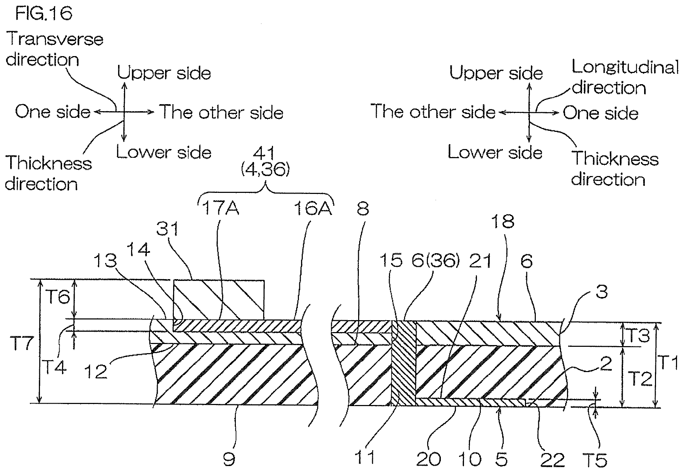

As shown in FIG. 16, the connecter 6 can have a substantially pillar shape, and the probe 5 can have no hole but a substantially plate shape (to be specific, substantially disc shape) extending in the surface direction.

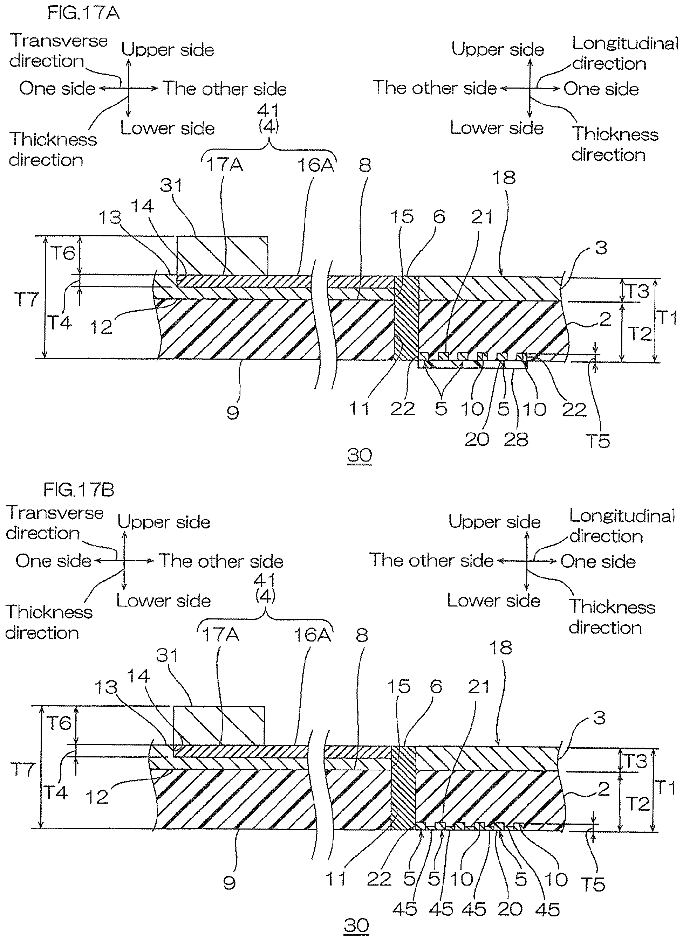

As shown in FIG. 17A, an electrically conductive pressure-sensitive adhesive layer 28 can be provided at the lower face of the probe 5.

The electrically conductive pressure-sensitive adhesive layer 28 is provided for suppressing reduction in sensing precision and suppressing noises based on various moisture content of the skin 33 and various bumps and dips of the surface of the living body (individuals), and it can have moisture content adjustment function (or moisture content stabilizing function) for adjusting the moisture content of the skin 33.

The material of the electrically conductive pressure-sensitive adhesive layer 28 can also be a material that has electrical conductivity and has moisture content adjustment function (or moisture content stabilizing function) (for example, hydrophilic compound, etc.). Examples of such materials include a composition in which silicone, acrylic, or urethane pressure-sensitive adhesive is blended with a hydrophilic polymer (hydrophilic compound) such as polyethylene oxide (PEO), polyvinyl alcohol (PVA), polyvinyl pyrrolidone (PVP), and polyethylene glycol (PEG). These materials are applied, for example, thereby providing the electrically conductive pressure-sensitive adhesive layer 28.

As shown in FIG. 17B, a strong pressure-sensitive adhesive layer 45 can be charged in the hole of the probe 5. The lower face of the strong pressure-sensitive adhesive layer 45 is flush with the probe lower face 20 and the adhesive lower face 9. For the material of the strong pressure-sensitive adhesive layer 45, for example, silicone, acrylic, or urethane strong pressure-sensitive adhesive is used. The peel strength of the strong pressure-sensitive adhesive layer 45 is, for example, 1.5 times the peel strength of the pressure-sensitive adhesive layer 2. The strong pressure-sensitive adhesive layer 45 allows the probe 5 to be strongly fixed to the skin 33, and therefore the signal can be processed with more precision.

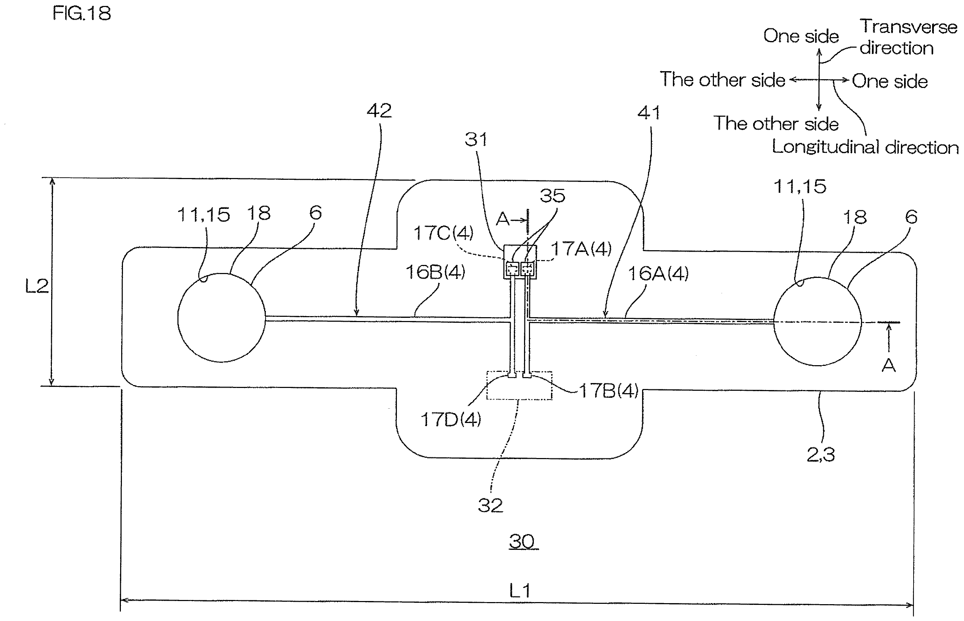

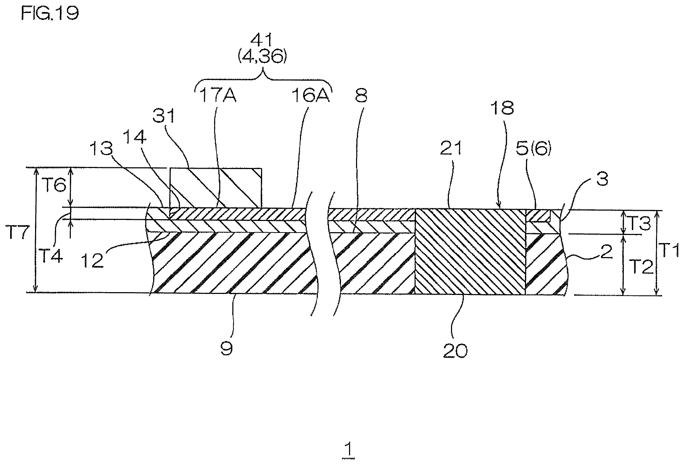

As shown in FIGs. 18 and 19, the probe 5 and the connecter 6 can be integrated.

That is, the probe 5 also works as the connecter 6. The probe 5 has a solid cylindrical shape. The probe lower face 20 is exposed from the adhesive lower face 9. The probe upper face 21 is exposed from the substrate upper face 13. The upper end portion of the two probes 5 is in contact with the longitudinal one end edge of the first wire 16A and the longitudinal other end edge of the second wire 16B. For the material of the probe 5, for example, the same materials as those of the connecter 6 are used.

Although not shown, the circuit portion 36 can be a circuit portion 36 integrally including the wire layer 4 and the connecter 6.

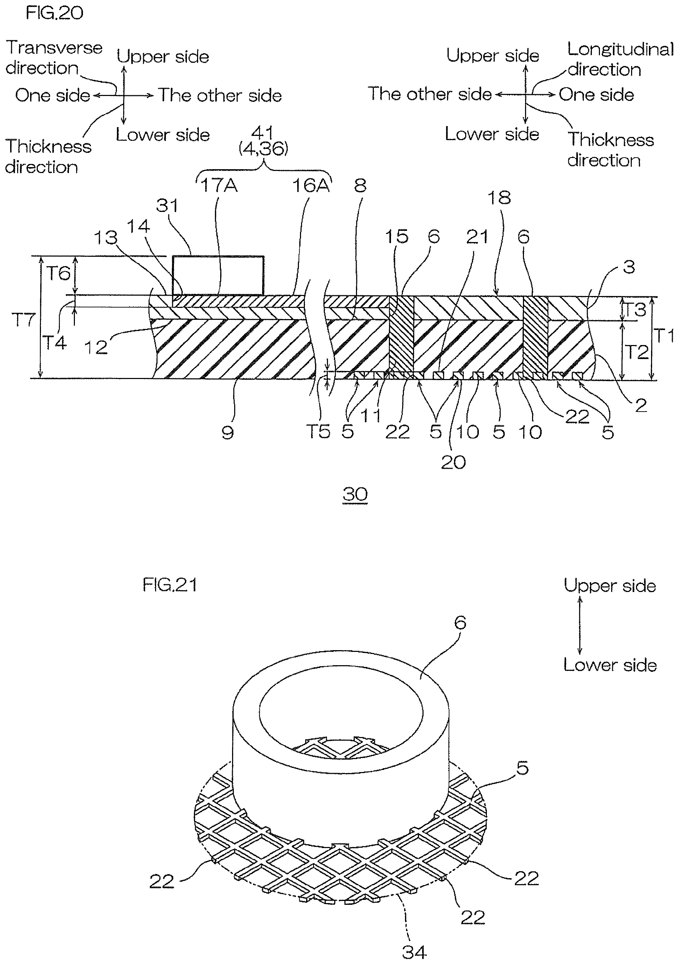

As shown in FIGs. 20 and 21, the connecter 6 can be smaller than the probe 5 in plan view. The virtual circle 34 passing through the outer side face 22 of the probe 5 includes the connecter 6 and is larger than the connecter 6 in plan view. The lower end edge of the connecter 6 is in contact with the middle portion (inner side than the outer side face 22) in the surface direction of the probe 5.

As shown in FIG. 1, in the embodiment, the longitudinal center portion of the wearable biosensor 30 bulges, but it is not limited thereto, and for example, although not shown, the wearable biosensor 30 can have a substantially rectangular shape in plan view without the bulging longitudinal center portion.

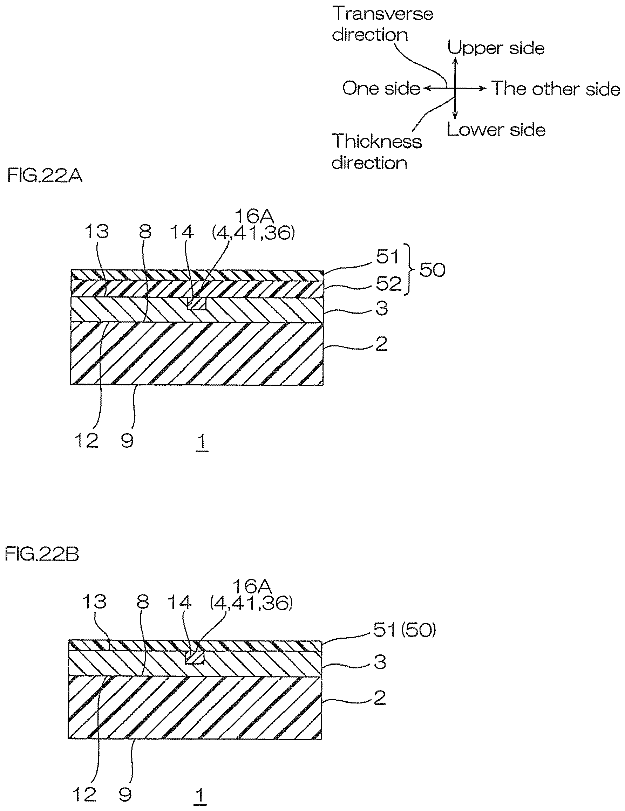

As shown in FIG. 22A and FIG. 22B, the biosensor laminate 1 can further include a protection member 50.

The protection member 50 is positioned at the upper end portion of the biosensor laminate 1.

To be specific, the protection member 50 is disposed at the substrate upper face 13 of the substrate layer 3 so as to close the upper face of the wire layer 4. The protection member 50 has a sheet shape along the substrate upper face 13. Therefore, the protection member 50 is a protection layer (upper face protection layer) that protects the substrate upper face 13 and the upper face of the wire layer 4.

As shown in FIG. 22A, the protection member 50 includes, for example, a protection substrate 51 and a second pressure-sensitive adhesive layer 52. The protection member 50 includes, in sequence toward the lower side, a protection substrate 51 and a second pressure-sensitive adhesive layer 52. In the modified example shown in FIG. 22A, the protection member 50 is composed only of the protection substrate 51 and second pressure-sensitive adhesive layer 52.

The second pressure-sensitive adhesive layer 52 is in contact with the substrate upper face 13 and the upper face of the wire layer 4. Examples of the material for the second pressure-sensitive adhesive layer 52 include an acrylic pressure-sensitive adhesive, silicone pressure-sensitive adhesive, polyolefin pressure-sensitive adhesive, and epoxy pressure-sensitive adhesive, and preferably, in view of achieving excellent moisture permeability, an acrylic pressure-sensitive adhesive is used. The second pressure-sensitive adhesive layer 52 has a thickness of, for example, 1 µm or more, and for example, 50 µm or less.

The protection substrate 51 is disposed at the upper face of the second pressure-sensitive adhesive layer 52. The protection substrate 51 has a sheet shape along the upper face of the second pressure-sensitive adhesive layer 52. The protection substrate 51 is in contact with the upper face of the second pressure-sensitive adhesive layer 52. In this manner, the protection substrate 51 is pressure-sensitively allowed to adhere to the substrate upper face 13 and the upper face of the wire layer 4 through the second pressure-sensitive adhesive layer 52. Examples of the material of the protection substrate 51 include resin. Examples of the resin include thermoplastic resin such as polyurethane resin, silicone resin, acrylic resin, polystyrene resin, vinyl chloride resin, and polyester resin, and in view of securing excellent stretching property, preferably, polyurethane resin is used. The protection substrate 51 has a thickness of, for example, 0.1 µm or more, and for example, 50 µm or less.

The protection member 50 has a thickness of a total of the thickness of the protection substrate 51 and the thickness of the second pressure-sensitive adhesive layer 52, and for example, 1.1 µm or more, and for example, 51 µm or less.

As shown in FIG. 22B, the protection member 50 can be composed of only the protection substrate 51 without including the second pressure-sensitive adhesive layer 52. The protection substrate 51 is disposed directly on the substrate upper face 13 and the upper face of the wire layer 4, and to be specific, is in contact with them.

While the illustrative embodiments of the present invention are provided in the above description, such is for illustrative purpose only and it is not to be construed as limiting in any manner. Modification and variation of the present invention that will be obvious to those skilled in the art is to be covered by the following claims.

### Industrial Applicability

The biosensor is used, for example, for a wearable biosensor.

### Description of Reference Numerals

2 pressure-sensitive adhesive layer
3 substrate layer
8 adhesive upper face (an example of pressure-sensitive adhesive layer upper face)
9 adhesive lower face (an example of pressure-sensitive adhesive layer lower face)
30 wearable biosensor
31 electronic component
T1 total thickness of pressure-sensitive adhesive layer and substrate layer
T2 total thickness of pressure-sensitive adhesive layer
T6 electronic component thickness
S flat area of electronic component

## Claims

1. A biosensor comprising:
a pressure-sensitive adhesive layer for attaching to a surface of a living body,
a substrate layer disposed on an upper face of the pressure-sensitive adhesive layer and having a stretching property,
a probe disposed on the lower face of the pressure-sensitive adhesive layer, and
an electronic component mounted on the substrate layer so as to be connected to the probe,
wherein
a total thickness of the pressure-sensitive adhesive layer and the substrate layer is 1 µm or more and less than 100 µm.

2. The biosensor according to Claim 1, wherein
the pressure-sensitive adhesive layer has a thickness of 10 µm or more and 95 µm or less.

3. The biosensor according to Claim 1, wherein
the electronic component has a thickness of 1 µm or more and 1000 µm or less.

4. The biosensor according to Claim 1, wherein
the electronic component has a flat area of 0.001 mm² or more and 10 mm² or less.
